# Europäisches Patentamt

## European Patent Office

## Office européen des brevets

(19)

(11) Veröffentlichungsnummer: **0 151 440**
**B1**

(12) # EUROPÄISCHE PATENTSCHRIFT

(45) Veröffentlichungstag der Patentschrift:
**17.11.88**

(51) Int. Cl.⁴: **C 07 D 201/08**

(21) Anmeldenummer: **85100741.9**

(22) Anmeldetag: **25.01.85**

(54) Verfahren zur Gewinnung von Caprolactam aus Eta-Aminocapronsäure.

(30) Priorität: **02.02.84 DE 3403574**

(43) Veröffentlichungstag der Anmeldung:
**14.08.85 Patentblatt 85/33**

(45) Bekanntmachung des Hinweises auf die Patenterteilung:
**17.11.88 Patentblatt 88/46**

(84) Benannte Vertragsstaaten:
**BE CH DE IT LI NL**

(56) Entgegenhaltungen:
**DE - A - 1 944 910**
**FR - A - 1 184 282**

**TETRAHEDRON LETTERS, Band 21, 1980, Seiten 2443-2446, Pergamon Press Ltd., GB; A. BLADE-FONT: "Facile synthesis of gamma-, delta-, and epsilon-lactams by cyclodehydration of omega-amino acids on alumina or silica gel"**

Die Akte enthält technische Angaben, die nach dem Eingang der Anmeldung eingereicht wurden und die nicht in dieser Patentschrift enthalten sind.

(73) Patentinhaber: **BASF Aktiengesellschaft, Carl-Bosch-Strasse 38, D-6700 Ludwigshafen (DE)**

(72) Erfinder: **Fuchs, Hugo, Dr., Egellstrasse 28, D-6700 Ludwigshafen (DE)**

## Beschreibung

Bei der Herstellung von Polycaprolactam erhält man zunächst Polycaprolactam mit einem Gehalt an Monomeren und Oligomeren von etwa 10 Gew.-%. Aus dem Monomeren enthaltenden Polycaprolactam werden die Monomeren und die Oligomeren nach bekannten Verfahren durch Wasser extrahiert. Aus solchen wässrigen Extrakten wird Caprolactam durch Extraktion mit Lösungsmitteln oder durch Destillation zurückgewonnen. Es verbleiben jedoch erhebliche Mengen an behandlungsbedürftigen Rückständen, die u.a. erhebliche Mengen an Aminocapronsäure enthalten.

Aus Tetrahedron Letters 1980, Band 21, Seiten 2443 bis 2446 ist ein Verfahren zur Herstellung von Caprolactam durch Behandeln von Aminocapronsäure in Gegenwart von Aluminiumoxid unter fortwährendem Entfernen des hierbei entstehenden Wassers bekannt. Dieses Verfahren ist technisch insofern unzulänglich, als es erhebliche Zeit z.B. 20 Stunden erfordert. Nach einem anderen aus der DE-OS 1 944 910 bekannten Verfahren erhält man Caprolactam durch Behandeln von ε-Aminocapronsäure mit überhitztem Wasserdampf bis zu 6 Stunden bei Temperaturen von 200 bis 350 °C in Gegenwart von nichtflüchtigen sauren Katalysatoren, z.B. Phosphorsäuren und deren sauren Salze, Schwefelsäure und deren sauren Salze, Sulfonsäuren, saure Zeolithe, Aluminiumphosphat, Titanphosphat oder Borsäureanhydrid, in einer Menge von 0,1 bis 20 Gew.-% bezogen auf ε-Aminocapronsäure. Das nach dem bekannten Verfahren erhaltene Caprolactam ist noch stark verunreinigt und bedarf weiterer aufwendiger Reinigungsmassnahmen. Insbesondere hat das so erhaltene Caprolactam eine hohe Permanganattitrationszahl und UV-Kennzahl, so daß es nicht ohne weiteres mit Rohcaprolactam aufgearbeitet werden kann, ohne dessen Qualität erheblich zu vermindern. Es ist deshalb erforderlich, so erhaltenes Caprolactam getrennt zu reinigen, was technisch sehr aufwendig ist.

Es war deshalb die technische Aufgabe gestellt, die Herstellung von Caprolactam aus ε-Aminocapronsäure so zu gestalten, daß man hohe Ausbeuten in kurzer Zeit erzielt und zugleich Caprolactam in einer Qualität erhält, die es erlaubt, dieses ohne Nachteile zusammen mit Rohcaprolactam aufzuarbeiten.

Diese Aufgabe wird gelöst in einem Verfahren zur Gewinnung von Caprolactam aus ε-Aminocapronsäure durch Behandeln mit Wasserdampf bei erhöhter Temperatur in Gegenwart von Katalysatoren, wobei man ε-Aminocapronsäure in eine Aluminiumoxidwirbelschicht einbringt und unter Mitverwendung von Wasserdampf bei einer Temperatur von 290 bis 400 °C behandelt.

Das neue Verfahren hat den Vorteil, daß man Caprolactam in hohen Ausbeuten erhält. Weiterhin hat das neue Verfahren den Vorteil, daß es in kurzer Zeit verläuft und einfach durchzuführen ist. Ferner hat das neue Verfahren den Vorteil, daß man Caprolactam mit einer verringerten Permanganattitrationszahl und UV-Kennzahl erhält. Schließlich ist das neue Verfahren insofern bemerkenswert, als es nicht erforderlich ist, von reiner ε-Aminocapronsäure auszugehen, sondern es kann auch oligomeres Caprolactam enthaltende ε-Aminocapronsäure verwendet werden, wobei nach dem neuen Verfahren zugleich Oligomere des Caprolactams zu Caprolactam zurückgespalten werden.

Erfindungsgemäß geht man von ε-Aminocapronsäure aus. Zur besseren Handhabung ist es auch möglich, wäßrige Lösungen z.B. 5 bis 60gewichtsprozentige Lösungen von ε-Aminocapronsäure anzuwenden. Andererseits ist es möglich, feste ε-Aminocapronsäure zusammen mit Caprolactam, z.B. 80 bis 99 Gew.-% Caprolactam, zu verwenden. Es ist auch möglich, Destillationsrückstände von Extraktwässern des Polycaprolactams nach weitgehendem Abdestillieren von Caprolactam zu verwenden. Solche Destillationsrückstände enthalten neben ε-Aminocapronsäure sowohl Caprolactam als auch Oligomere des Caprolactams. Solche Oligomere haben in der Regel einen Polymerisationsgrad n von 2 bis 9. Insbesondere enthalten sie dimere und trimere cyclische Oligomere. Geeignete Gemische enthalten beispielsweise 0,1 bis 10 Gew.-% ε-Aminocapronsäure, 40 bis 95 Gew.-% Caprolactam und 5 bis 50 Gew.-% Oligomere.

Die oben genannten Gemische werden vorteilhaft in flüssiger Form, d.h. in geschmolzenem Zustand bzw. als Schmelzsuspension z.B. mit einer Temperatur von 150 bis 250 °C in ein Aluminiumoxidwirbelbett eingebracht. Es ist jedoch auch möglich, die ε-Aminocapronsäure oder solche enthaltende Gemische, wie vorgenannt, in fester, feinverteilter Form in die Wirbelschicht einzubringen und in monomeres Caprolactam umzusetzen. Das Einbringen in die Wirbelschicht erfolgt z.B. durch Einblasen mittels einer mit Inertgas betriebenen Düse.

Als Aluminiumoxid eignen sich die verschiedenen Modifikationen wie Tonerde oder Böhmit. Besonders bewährt hat sich γ-Aluminiumoxid als Katalysator. Der Katalysator wird mit Inertgas, wie Kohlendioxid, Argon oder Stickstoff, vorzugsweise Stickstoff, in wirbelnder Bewegung gehalten. Zweckmäßig verwendet man Aluminiumoxid in Korngrößen von 0,05 bis 1,5 mm, insbesondere von 0,2 bis 1 mm. Die Höhe der Katalysatorschicht wird vorteilhaft so gewählt, daß die Verweilzeit in der Katalysatorschicht von 0,1 bis 30, insbesondere von 0,5 bis 10 Sekunden beträgt. Vorteilhaft führt man das Verfahren bei Normaldruck durch. Es kann jedoch auch bei schwach vermindertem oder leicht erhöhtem Druck, z.B. bis zu 2 bar durchgeführt werden.

Die Katalysatorschicht wird auf einer Temperatur von 290 bis 400 °C gehalten. Besonders bewährt haben sich Temperaturen von 300 bis 360 °C. Es ist deshalb auch zweckmäßig, das Inertgas mit einer Temperatur von 290 bis 400 °C der Wirbelschicht zuzuführen.

Erfindungsgemäß führt man die Behandlung unter Mitverwendung von Wasserdampf durch. Vorteilhaft verwendet man je Gewichtsteil ε-Aminocapronsäure 0,005 bis 10 Gewichtsteile, insbesondere 0,02 bis 2 Gewichtsteile Wasser in Form von Wasserdampf. Falls die Ausgangsgemische wie vorgenannt Oligomere enthalten, beziehen sich die angegebenen Wassermengen auf die Summe aus ε-Aminoca-

pronsäure und Oligomere. Das mitzuverwendende Wasser kann als solches in die Wirbelschicht eingebracht und dort verdampft werden. Vorzugsweise führt man das Wasser jedoch in Form von Wasserdampf zu. Beispielsweise kann dieser auch zusammen mit dem Inertgas in die Wirbelschicht eingeführt werden.

Das aus der Wirbelschicht austretende Gasgemisch wird wie beispielsweise in der DE-AS 14 45 549 beschrieben, in einer Glockenbodenkolonne durch Aufgabe von Wasser auf den Kopf der Kolonne kondensiert. Als Sumpfprodukt erhält man Caprolactam. Über den Kopf der Kolonne entweichen das Inertgas sowie Wasserdampf. Der Wasserdampf wird vorteilhaft aus dem Inertgas kondensiert und das Inertgas zweckmässig wieder in die Wirbelschicht zurückgeführt.

Das kondensierte Caprolactam wird zweckmäßig z.B. durch Destillation nochmals gereinigt und anschließend das so zurückgewonnene Caprolactam dem zu reinigenden Caprolactam aus der Beckmannschen Umlagerung zugesetzt und zusammen aufgearbeitet, z.B. nach dem in der DE-PS 1 194 863 beschriebenen Verfahren.

Es ist aber auch möglich, das Caprolactam aus dem aus der Wirbelschicht austretenden Dämpfegemisch, wie beschrieben, zu kondensieren und dieses direkt dem Rohlactam aus der Beckmannschen Umlagerung zuzufügen und gemeinsam aufzuarbeiten.

Caprolactam wird für die Herstellung von Polycaprolactam verwendet. Das Verfahren nach der Erfindung sei in folgenden Beispielen veranschaulicht.

Beispiel 1

In einem senkrecht stehenden, isolierten, elektrisch beheizten, unten mit einem Lochboden versehenen Rohr von 1200 mm Länge und 100 mm Durchmesser werden 1000 g Katalysator, bestehend aus einem bei 800 °C geglühten γ-Aluminiumoxid der Korngröße 0,2 bis 0,8 mm, auf 320 °C erhitzt.

Durch einen von unten durch den Lochboden eingeblasenen auf 360 °C vorgewärmten Stickstoffstrom von 2500 Nl/h wird der Katalysator zum Wirbeln gebracht. Durch eine 90 mm oberhalb des Lochbodens zentrisch im Rohr befindliche, nach oben gerichtete Zweistoffdüse wird mit Hilfe eines Stickstoffstromes von 1500 Nl/h, der auf 200 °C vorgeheizt wurde, aus einem auf 170 °C warmen Vorratsgefäß im Laufe einer Stunde 4500 g einer Caprolactam-ε-Aminocapronsäure-Oligomerenschmelze bzw. Schmelzsuspension mit 5% m/m ε-Aminocapronsäure und 30% m/m Oligomeren in die Katalysatorschicht eingedüst. Dem vorgeheizten Stickstoffstrom werden stündlich 600 g Wasserdampf zugefügt. Die Temperatur im Katalysatorbett wird durch die elektrische Heizung bei 320 °C gehalten. Die den Reaktor verlassenden Dämpfe werden in einer Glockenbodenkolonne vom Durchmesser 100 mm, mit 10 Böden durch Aufgabe von Wasser auf den Kolonnenkopf kondensiert. Man erhält so stündlich 4300 g Caprolactam, welches aminocapronsäure- und nahezu oligomerenfrei ist. Die Kennzahlen des erhaltenen Lactams, bezogen auf wasserfreies Lactam, sind folgende:

PTZ 180     UV-Kennzahl 520

500 g dieses Lactams werden zu 5000 g Extraktlactam mit folgenden Kennzahlen PTZ 50    UV-Kennzahl 110    fl. Basen 0,6 meq/kg gegeben und gemeinsam durch Destillation im Vakuum zu Reinlactam aufgearbeitet.

Das erhaltene Reinlactam weist folgende Kennzahlen auf:

| | |
|---|---|
| Erstarrungspunkt | 69,1 °C |
| PTZ | 1,5 |
| PAZ | 3,8 |
| UV-Kennzahl | 4,8 |
| Extinktion bei 290 nm in 1 cm Schichtdicke (50% wäßrige Lösung) | 0,03 |
| fl. Basen | 0,15 meq/kg |

Beispiel 2

In einer Apparatur, wie in Beispiel 1 beschrieben, werden 1000 g γ-Aluminiumoxid als Katalysator auf 300 °C erhitzt und mit 2500 Nl/h Stickstoff zum Wirbeln gebracht. Über eine Düse, die mit etwa 2000 Nl/h Stickstoff betrieben wird, werden stündlich ein Gemisch aus 200 g Oligomere und 200 g ε-Aminocapronsäure in Pulverform in die heiße Katalysatorschicht gebracht. Mit dem zum Wirbeln notwendigen Stickstoff werden gleichzeitig 2000 g Wasserdampf in die Katalysatorschicht gegeben. Das den Reaktor verlassende Dämpfegemisch wird wie beschrieben kondensiert.

Stündlich werden 350 g monomeres Caprolactam mit folgenden Zahlen erhalten:

| | |
|---|---|
| PTZ | 300 |
| UV-Kennzahl | 400 |

250 g dieses Lactams werden mit 2500 g Extraktlactam zusammen, wie beschrieben, zu Reinlactam aufgearbeitet. Die Kennzahlen des Reinlactams sind folgende:

| | |
|---|---|
| Erstarrungspunkt | 69,12 °C |
| PTZ | 1,6 |
| PAZ | 4,2 |
| UV-Kennzahl | 3,55 |
| Extinktion bei 290 mm in 1 cm Schichtdicke (50% wäßrige Lösung) | 0,025 |
| fl. Basen | 0,11 meq/kg |

Beispiel 3

In einer Apparatur, wie in Beispiel 1 beschrieben, werden 1000 g γ-Aluminiumoxid als Katalysator auf 300 °C erhitzt und mit 2500 Nl/h Stickstoff zum Wirbeln gebracht. Über eine Düse werden stündlich 1000 g einer 40% m/m wäßrigen ε-Aminocapronsäurelösung in die heiße Katalysatorschicht eingedüst. Das den Reaktor verlassende Dämpfgemisch wird, wie beschrieben, kondensiert. Aus der erhaltenen wäßrigen Caprolactamlösung werden nach Entfernen des Wassers stündlich 304 g Caprolactam gewonnen, welches folgende Zahlen aufwies:

| | |
|---|---|
| PTZ: | 270 |
| UV-Kennzahl | 520 |

Das erhaltene Lactam ließ sich zusammen mit Extraktlactam, wie beschrieben, zu einem spezifikationsgerechten Reinlactam aufarbeiten.

UV-Zahl

Prinzip:

Im Spektralbereich von 360 bis 270 nm wird die Extinktion des Caprolactams gemessen und nach Umsetzung in einer Kennzahl ausgedrückt.

Analysengeräte:

1 registrierendes Spektralphotometer

1 Erlenmeyerkolben (200 ml)

2 Quarz-Küvetten mit Deckel, 10 cm lang (Schichtdicke 10 cm)

Vorschrift:

50 g Caprolactam werden in einem Erlenmeyerkolben in 50 g bidestilliertem Wasser kalt aufgelöst. Mit dieser Lösung wird eine Küvette bis zur Eichmarke gefüllt. Die zweite Küvette wird mit dem gleichen bidestillierten Wasser gefüllt und stellt die Vergleichslösung dar.

Nun werden beide Küvetten mit den Deckeln verschlossen, die geschliffenen Flächen mit Seidenpapier gereinigt und in die Küvettenhalter eingesetzt. Dann wird gemäß Geräteanleitung das Spektrum zwischen 370 nm und 260 nm aufgenommen. Die Registriergeschwindigkeit beträgt 50. Die Extinktionsmessung wird im Meßbereich 0 bis 1 ausgeführt.

Ist die Aufnahme beendet, wird von 270 bis 360 nm alle 10 nm eine Markierung auf dem Papier angebracht.

Auswertung:

Aus dem Diagramm werden die Extinktionen bei 270, 280, 290, 300, 310, 320, 330, 340, 350 und 360 nm abgelesen und addiert.

Die Summe der 10 Extinktionswerte wird mit 2 multipliziert und ergibt die UV-Kennzahl. Die UV-Kennzahl wird also immer auf 100%iges Caprolactam und auf eine Schichtdicke von 10 cm bezogen.

Flüchtige Basen

Zur Bestimmung der flüchtigen Basen werden in einer Kjeldahl-Apparatur 40 ml 30%ige Natronlauge und danach eine Lösung von 20 g Substanz in 80 ml destilliertes Wasser eingefüllt. Man spült mit 20 ml destilliertem Wasser nach. Dann destilliert man unter Einblasen von Wasserdampf in einer Zeit von 5 Minuten 50 ml Wasser in die Vorlage über, die 5 ml n/50 Salzsäure, 30 ml Wasser und 5 Tropfen Indikatorlösung enthält. Danach spült man den Kühler samt Ablaufrohr mit destilliertem Wasser in die Vorlage aus und titriert mit n/50 Natronlauge die nicht verbrauchte Säure zurück. Unter Berücksichtigung des Blindversuchs gibt der Verbrauch an n/50 Salzsäure unmittelbar die flüchtigen Basen in mäq Basen je kg Substanz.

PTZ

Unter der «Permanganat-Titrationszahl», abgekürzt «PTZ», ist der Verbrauch an n/10 Kaliumpermanganatlösung in Millilitern zu verstehen, den eine Lösung von 1000 g Substanz in 2500 g 50%iger wäßriger Schwefelsäure hat, bis bei Titration bei Raumtemperatur die Permanganatfarbe 2 Minuten beständig ist.

PAZ = Permanganat-Absorptionszahl

Gemessen wird die Extinktion einer 1%igen Caprolactamlösung in Wasser (50 ml bzw. 100 ml Lösung) nach Zugabe von 0,01 n $KMnO_4$-Lösung (1 bzw. 2 ml) bei 25 °C gegen eine gleiche Lösung ohne Caprolactam nach 600 sec. bei 420 nm.

## Patentansprüche

1. Verfahren zur Gewinnung von Caprolactam aus ε-Aminocapronsäure durch Behandeln mit Wasserdampf bei erhöhter Temperatur in Gegenwart von Katalysatoren, dadurch gekennzeichnet, daß man ε-Aminocapronsäure in eine Aluminiumoxidwirbelschicht einbringt und unter Mitverwendung von Wasserdampf bei einer Temperatur von 290 bis 400 °C behandelt.

2. Verfahren nach Anspruch 1, dadurch gekennzeichnet, daß man 0,005 bis 10 Gewichtsteile Wasser in Form von Wasserdampf je Gewichtsteil ε-Aminocapronsäure mitverwendet.

3. Verfahren nach den Ansprüchen 1 und 2, dadurch gekennzeichnet, daß man eine Temperatur von 300 bis 360 °C einhält.

4. Verfahren nach den Ansprüchen 1 bis 3, dadurch gekennzeichnet, daß man ε-Aminocapronsäure, das zusätzlich Caprolactam und/oder Oligomere der Caprolactams enthält, verwendet.

5. Verfahren nach den Ansprüchen 1 bis 4, dadurch gekennzeichnet, daß man γ-Aluminiumoxid als Katalysator verwendet.

## Claims

1. A process for obtaining caprolactam from ε-aminocaproic acid by treatment with steam at elevated temperatures in the presence of a catalyst, which comprises introducing ε-aminocaproic acid into a fluidized alumina bed and treating it in the presence of steam at from 290 to 400 °C.

2. A process as claimed in claim 1, wherein from 0.005 to 10 parts by weight of water in the form of steam are used per part by weight of ε-aminocaproic acid.

3. A process as claimed in claims 1 and 2, wherein the temperature is kept at from 300 to 360 °C.

4. A process as claimed in an of claims 1 to 3, wherein the ε-aminocaproic acid used additionally contains caprolactam and/or its oligomers.

5. A process as claimed in any of claims 1 to 4, wherein γ-alumina is used as the catalyst.

## Revendications

1. Procédé de préparation de caprolactame à partir de l'acide ε-amino-caproïque par traitement à la vapeur d'eau à température accrue en présence de catalyseurs, caractérisé en ce que l'on introduit l'acide ε-amino-caproïque dans un lit fluidisé d'oxyde d'aluminium et on l'y traite à la vapeur d'eau à une température comprise entre 290 et 400 °C.

2. Procédé suivant la revendication 1, caractérisé en ce que, pour chaque partie en poids d'acide ε-amino-caproïque, on utilise entre 0,005 et 10 parties en poids d'eau à l'état de vapeur.

3. Procédé suivant les revendications 1 et 2, caractérisé en ce que la température est maintenue entre 300 et 360 °C.

4. Procédé suivant les revendications 1 à 3, caractérisé en ce que l'on utilise de l'acide ε-amino-ca-proïque, qui contient encore de la caprolactame et (ou) des oligomères de la caprolactame.

5. Procédé suivant les revendications 1 à 4, caractérisé en ce que l'on emploie comme catalyseur de l'oxyde d'aluminium γ.